# EUROPÄISCHE PATENTSCHRIFT

(11) **EP 2 748 157 B1**
(45) Veröffentlichungstag und Bekanntmachung des Hinweises auf die Patenterteilung: **07.10.2015**
(21) Anmeldenummer: 12751327.3
(22) Anmeldetag: 24.08.2012
(51) Int. Cl.: C07D 401/14, C07D 405/14

(54) **VERFAHREN ZUR HERSTELLUNG VON TETRAZOL-SUBSTITUIERTEN ANTHRANILSÄUREDIAMID-DERIVATEN DURCH UMSETZUNG VON BENZOXAZINONEN MIT AMINEN**
METHOD FOR PRODUCING TETRAZOLE-SUBSTITUTED ANTHRANILIC ACID DIAMIDE DERIVATIVES BY REACTING BENZOXAZINONES WITH AMINES
PROCÉDÉ DE PRODUCTION DE DÉRIVÉS DE DIAMIDE D'ACIDE ANTHRANILIQUE SUBSTITUÉS PAR DU TÉTRAZOL PAR LA MISE EN RÉACTION DE BENZOXAZINONES ET D'AMINES

(30) Priorität: 26.08.2011 EP 11179028; 26.08.2011 US 201161527645 P
(43) Veröffentlichungstag der Anmeldung: 02.07.2014
(73) Patentinhaber: Bayer Intellectual Property GmbH, 40789 Monheim (DE)
(72) Erfinder: PAZENOK, Sergii, 42699 Solingen (DE); LINDNER, Werner, 51067 Köln (DE); SCHEFFEL, Hartmut, 42281 Wuppertal (DE)
(74) Vertreter: BIP Patents
(86) Internationale Anmeldenummer: PCT/EP2012/066477
(87) Internationale Veröffentlichungsnummer: WO 2013/030100

(56) Entgegenhaltungen:
- WO-A1-2006/068669

## Beschreibung

Die vorliegende Erfindung betrifft ein Verfahren zur Herstellung von tetrazol-substituierten Anthranilsäurediamid-Derivaten der Formel (I) 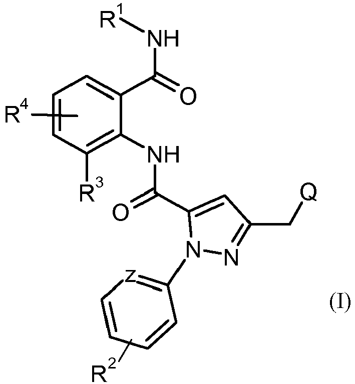 durch Umsetzung von Benzoxazinonen mit Aminen.

In der Literatur wurde bereits beschrieben, dass tetrazol-substituierte Anthranilsäurediamid-Derivate durch Umsetzung von tetrazol-substituierten N-aryl- und N-hetarylsubstituierten Pyrazolsäuren mit Anthranilsäureamiden hergestellt werden können (vgl. WO2010/069502). Ebenfalls können tetrazol-substituierte Anthranilsäurediamid-Derivate durch Umsetzung von tetrazol-substituierten Benzoxazinonen mit Aminen erhalten werden (WO 2010/069502). Die Umsetzung von Benzoxazinonen mit Aminen verläuft bis zu einem Umsatz von 90-95% selektiv, aber am Ende der Reaktion reagiert das tetrazol-substituierte Anthranilsäurediamid der Formel (I) unter basischen Bedingungen unter der Bildung von 4-oxo-3,4-dihydroquinazolinen der Formel (X). Dadurch wird die Reinheit des Produktes und Wirtschaftlichkeit des Verfahrens negativ beeinflusst.

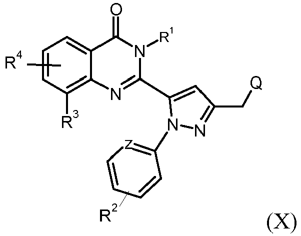

Die Aufgabe der vorliegenden Erfindung ist daher die Bereitstellung neuer, wirtschaftlicher Verfahren zur Herstellung von tetrazol-substituierten Anthranilsäurediamid-Derivaten der Formel (I) in höherer Reinheit.

Die Aufgabe wurde gemäß der vorliegenden Erfindung gelöst durch ein Verfahren zur Herstellung von Anthranilsäurediamid-Derivaten der allgemeinen Formel (I) 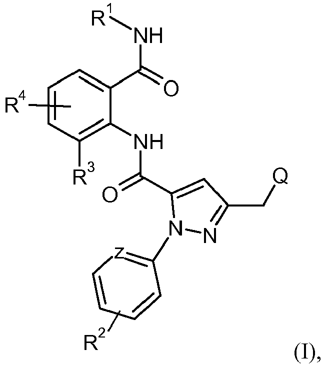 in welcher
- R¹,R³: unabhängig voneinander für Wasserstoff, für gegebenenfalls einfach oder mehrfach, gleich oder verschieden durch Halogen oder Nitro substituiertes C₁-C₆-Alkyl, C₁-C₆-Alkoxy, C₂-C₆-Alkenyl, C₂-C₆-Alkinyl oder C₃-C₆-Cycloalkyl steht, bevorzugt für (C₁-C₅)-Alkyl, besonders bevorzugt für Methyl, Ethyl oder tert-Butyl steht, ganz besonders bevorzugt für Methyl steht,
- R²: für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalkenyl, C₂-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro, Alkylamino, Dialkylamino, Cycloalkylamino oder C₃-C₆-Trialkylsilyl steht, bevorzugt für Halogen oder C₁-C₆-Alkyl steht, besonders bevorzugt für Fluor oder Chlor steht, ganz besonders bevorzugt für Chlor steht,
- R⁴: für Wasserstoff, Halogen, Cyano, Nitro, C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, SF₅, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₄-Alkoxy)imino, (C₁-C₄-Alkyl)(C₁-C₄-Alkoxy)imino, (C₁-C₄-Haloalkyl)(C₁-C₄-Cyano, Nitro, Alkoxy)imino oder C₁-C₆-Trialkylsilyl steht, bevorzugt für Wasserstoff, Chlor oder Cyano, besonders bevorzugt für Chlor oder Cyano steht, ganz besonders bevorzugt für Cyano steht,
- Q: für einen durch R⁵ einfach substituierten Tetrazolring steht, bevorzugt für einen durch R⁵ einfach substituierten Tetrazolring steht, ausgewählt aus der Gruppe bestehend aus 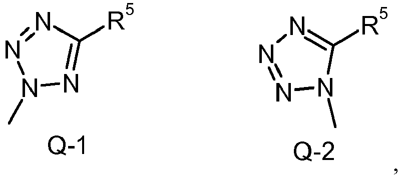 besonders bevorzugt für Q-1 steht, ebenfalls besonders bevorzugt für Q-2 steht,
- R⁵: für C₁-C₅-Alkyl, welches 1 bis 3 fach durch Halogen substituiert sein kann, bevorzugt für C₁-C₃ Perfluoralkyl, besonders bevorzugt für CF₃ oder C₂F₅ steht, ganz besonders bevorzugt für CF₃ steht,
- Z: für CH und N steht, bevorzugt für N steht,
die Verbindungen der allgemeinen Formel (I) außerdem N-Oxide und Salze umfassen,
dadurch gekennzeichnet, dass man tetrazol-substituierte Pyrazolsäuren der Formel (II) 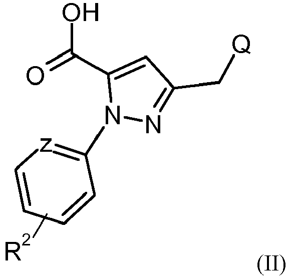 in welcher R², Q und Z oben angegebenen Bedeutungen haben
mit Isatoanhydriden der Formel (III) 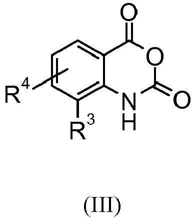 in welcher
R³,R⁴ die oben angegebenen Bedeutungen haben,
zu Verbindungen der Formel (IV) umsetzt 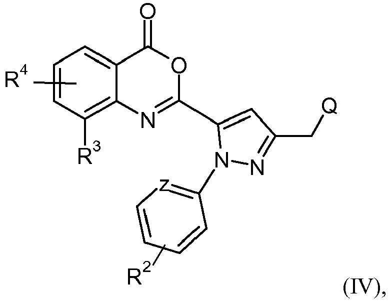 in welcher R², R³, R⁴, Q und Z oben angegebenen Bedeutungen haben,
und diese Verbindungen der allgemeinen Formel (IV) in Gegenwart einer Säure mit Aminen der allgemeinen Formel (V)

R¹NH₂ (V)

in welcher R¹ die oben angegebenen Bedeutungen hat,
umsetzt zu Anthranilsäurediamid-Derivaten der Formel (I), 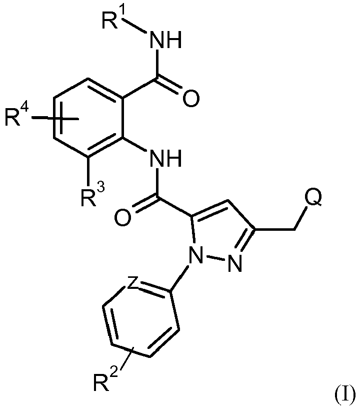 in welcher R¹, R², R³, R⁴, Q und Z die oben angegebenen Bedeutungen haben.

Durch das erfinderische Verfahren werden die Verbindungen der Formel (I) mit einer Reinheit von > 93 %, bevorzugt von 94 %-98 %, besonders bevorzugt von 95 % bis 97 % erhalten, wobei das IsomerenVerhältnis der beiden möglichen Regioisomeren mit 90:10 bis 96:4 (Hauptisomer A, wobei Q für Q-1 steht: Nebenisomer B, wobei Q für Q-2 steht) konstant bleibt.

### Hauptisomer A

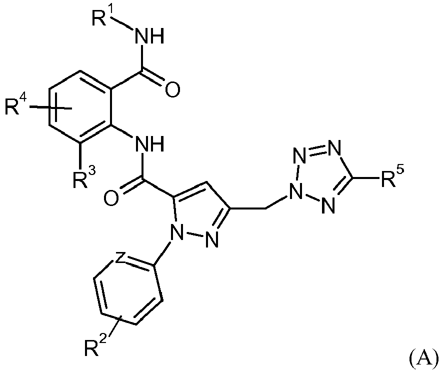

### Nebenisomer B

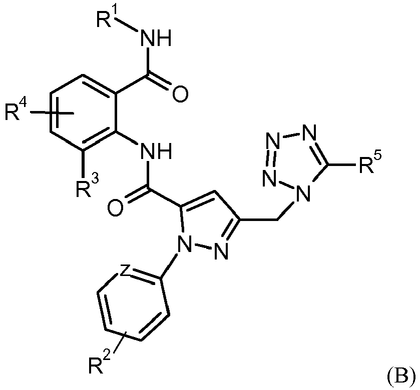

Das erfindungsgemäße Verfahren kann anhand des folgenden Schemas (I) erläutert werden : 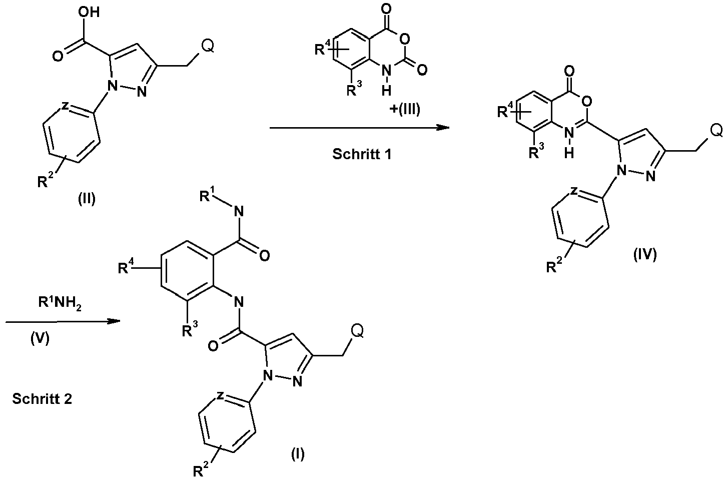 wobei R¹, R², R³, R⁴, Q und Z die oben angegebenen allgemeinen Bedeutungen haben.

### Schema (I)

### Allgemeine Definitionen:

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Jod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen (= Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. Alkoxy-Gruppen, die eine Alkylenkette aufweisen können. Im Einzelnen umfasst die Definition Arylalkyl beispielsweise die Bedeutungen Benzyl- und Phenylethyl-; die Definition Arylalkoxy beispielsweise die Bedeutung Benzyloxy.

Alkylaryl-Gruppen (Alkaryl-Gruppen) und Alkylaryloxy-Gruppen sind im Zusammenhang mit der vorliegenden Erfindung, soweit nicht abweichend definiert, durch Alkyl-Gruppen substituierte Aryl-Gruppen, bzw Aryloxy-Gruppen, die eine C₁₋₈-Alkylenkette aufweisen können und im Arylgerüst oder Aryloxygerüst ein oder mehrere Heteroatome, die ausgewählt sind aus O, N, P und S, aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart

### Schritt 1

2,4-dioxo-1,4-dihydro-2H-3,1-benzoxazine (Isatoanhydride) der Formel (III) sind bekannt (WO 2006/068669).

Die Verbindungen der Formel (IV) (Benzoxazinone) werden durch Umsetzung von tetrazolsubstituierten Pyrazolsäuren der Formel (II) mit Anthranilsäurederivaten der Formel (III) erhalten.

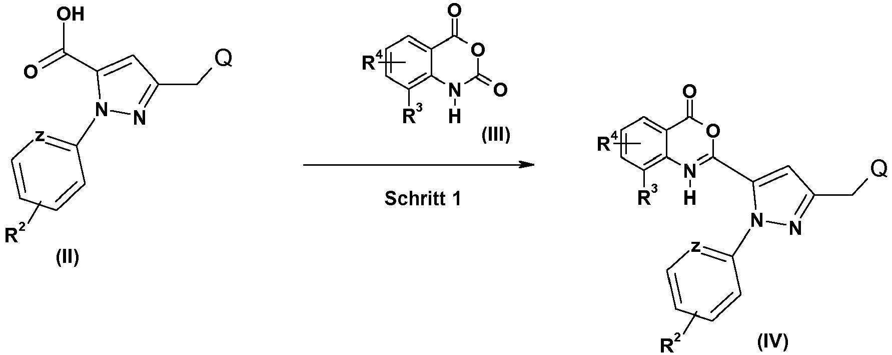

Pyrazolsäuren der Formel (II) sind bekannt (vgl. WO2007/144100). Pyrazolsäuren der Formel (II) können zum Beispiel aus Halogenmethylpyrazolestern der Formel (VI) und Perfluoralkyltetrazolen der Formel (VII) in zwei Schritten a und b hergestellt werden (vgl. Schema (II) und Herstellbeispiele). Dabei werden die gebildeten Verbindungen der Formel (VIII) durch basische Hydrolyse (Schritt b) in die Pyrazolsäuren der Formel (II) umgesetzt.

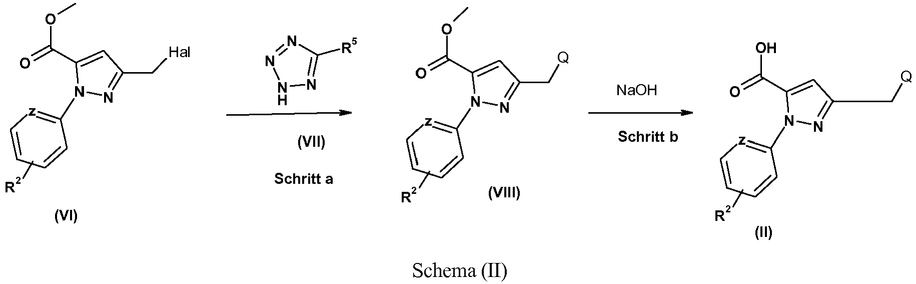

Halogenmethylpyrazolester der Formel (VI) sind ebenfalls bekannt und können wie in WO 2011/7073101 beschrieben hergestellt werden. Perfluoralkyltetrazole der Formel (VII) sind bekannt, teilweise sogar kommerziell erhältlich oder können nach bekannten Verfahren erhalten werden (vgl. z.B. WO2004/020445; William P. Norris, J. Org. Chem., 1962, 27 (9), 3248-3251; Henry C. Brown, Robert J. Kassal, J. Org. Chem., 1967, 32 (6), 1871-1873; Dennis P. Curran, Sabine Hadida, Sun-Young Kim, Tetrahedron, 1999, 55 (29), 8997-9006; L.D. Hansen, E.J. Baca, P. Scheiner, Journal of Heterocyclic Chemistry, 1970, 7, 991-996; JACS V.27, p.3248).

### Schritt 1

Schritt 1 wird grundsätzlich in Gegenwart einer Base durchgeführt. Geeignete Basen sind z. B. Natriumhydroxid, Kaliumcarbonat, Natriumcarbonat, Cäsiumcarbonat, Natriummethylat. Bevorzugt sind organischen Basen wie Trialkylamine, Pyridine, Alkylpyridine, Phosphazene und 1,8-Diazabicyclo[5.4.0]undecen (DBU). Besonders bevorzugt sind Pyridine, Alkylpyridine wie β-Picoline, 2,6-Dimethylpyridin, 2-Methyl-5-ethylpyridin, 2,3-Dimethylpyridin. Bei der Durchführung des erfindungsgemäßen Verfahrensschritts 1 setzt man für 1 Mol des Pyrazoles der Formel (II) vorzugsweise 1,5 Mol bis 4 Mol, besonders bevorzugt 1,5 bis 3 Äquivalente der Base ein. Schritt 1 wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid, p-Toluolsulphonsäure chlorid; Carbodiimide, wie N,N'-Dicyclohexylcarbodiimid (DCC) oder andere übliche Kondensationsmittel, wie Phosphorpentoxid, Polyphosphorsäure, 1,1'-Carbonyldiimidazol, 2-Ethoxy-N-ethoxycarbonyl-1,2-dihydrochinolin (EEDQ), Triphenylphosphin/Tetrachlorkohlenstoff, Brom-tripyrrolidinophosphonium-hexafluorophosphat, Bis(2-oxo-3-oxazolidinyl)phosphinchlorid oder Benzotriazol-1-yloxytris(dimethylamino)-phosphonium-hexafluorphos-phat. Auf Polymer gestützte Reagenzien, wie z.B. polymer-gebundenes Cyclohexylcarbodiimid, können ebenfalls verwendet werden. Besonders geeignet sind Methansulfonylchlorid (Mesylchlorid) und Phosgen. Bei der Durchführung des erfindungsgemäßen Verfahrensschritts 1 setzt man für 1 Mol des Pyrazoles der Formel (II) vorzugsweise 1 Mol bis 3 Mol, besonders bevorzugt 1,5 bis 2,5 Mol der Kondensationsmittels ein.

Die Durchführung des erfindungsgemäßen Verfahrensschritts erfolgt vorzugsweise innerhalb eines Temperaturbereichs von 0°C bis +80°C, besonders bevorzugt bei Temperaturen von 10°C bis +50 °C.

Bei der Durchführung des erfindungsgemäßen Verfahrensschritts setzt man für 1 Mol der Pyrazolsäure der Formel (II) eine äquimolare Menge der Verbindung der Formel (III) ein.

Der erfindungsgemäße Verfahrensschritt (1) wird im Allgemeinen unter Normaldruck durchgeführt. Alternativ ist es jedoch auch möglich, im Vakuum zu arbeiten oder unter Druck.

Die Reaktionszeit ist nicht kritisch und kann, in Abhängigkeit von der Ansatzgröße, vom Substituent R⁵ und von der Temperatur, in einem Bereich zwischen ein und mehreren Stunden gewählt werden. Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol. Besonders bevorzugt verwendet man Aceton, Acetonitril, Toluol, Methyl-tert-butylether, THF. Besonders geeignet sind Acetonitril, THF, DMF und NMP.

### Schritt 2

Die im Schritt 1 gebildeten Verbindungen der Formel (IV) werden zu Anthranilsäurediamid-Derivaten der Formel (I) umgesetzt:

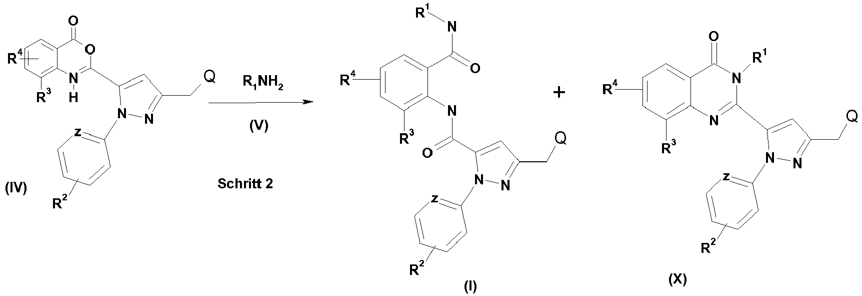

Überraschenderweise wurde nun gefunden dass die Verbindungen der Formel (IV) in Gegenwart einer Säure selektiv und unter sehr milden Bedingungen zu Anthranilsäurediamid-Derivaten der Formel (I) reagieren, wobei die Bildung von Nebenkomponenten, insbesondere die Bildung von 4-oxo-3,4-dihydroquinazoline-6-carbonitrile der Formel (X) weitgehend unterdrückt wird. Dabei wird die Reinheit des Produktes signifikant verbessert.

Unter sehr milden Bedingungen sind beispielsweise, aber nicht limitierend, die folgenden Bedingungen zu verstehen:

Die Reaktion wird im Allgemeinen unter Normaldruck durchgeführt. Alternativ ist es jedoch auch möglich, unter Überdrück (z.B. Umsetzung im Autoklav mit MeNH₂) zu arbeiten.

Die Reaktionszeit kann, in Abhängigkeit von der Ansatzgröße und der Temperatur, in einem Bereich zwischen 1 Stunde und mehreren Stunden gewählt werden.

Die Durchführung des erfindungsgemäßen Verfahrensschritts (2) erfolgt vorzugsweise innerhalb eines Temperaturbereichs von 0°C bis +100°C, besonders bevorzugt bei Temperaturen von 10°C bis +80 °C, besonders bevorzugt bei 10-60°C.

Der Reaktionsschritt wird vorzugweise in einem Lösungsmittel durchgeführt werden. Geeignete Lösungsmittel sind beispielsweise ausgewählt aus der Gruppe bestehend aus Wasser, Alkoholen wie Methanol, Ethanol, Isopropanol oder Buthanol, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Chlorbenzol oder Dichlorbenzol; Ethern, wie z.B. Diethylether, Diphenylether, Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wie Dimethylformamid (DMF) oder N-methylpyrrolidon (NMP) oder Mischungen solcher Lösungsmittel, wobei Wasser, Acetonitril, Dichlormethan und Alkohole (Ethanol) besonders gut geeignet sind. Besonders bevorzugt sind THF, Acetonitrile, Alkohole.

Die Amine der Formel (V) können als Substanz oder als Lösung in Wasser, THF, Acetonitril oder Alkoholen oder in einem anderen Lösemittel eingesetzt werden.

Man verwendet die Verbindungen der Formel (V) wobei R¹ bevorzugt für (C₁-C₆) Alkyl steht.

Geeigneten Säuren sind organische und anorganische Säuren wie HCOOH, CH₃COOH, CF₃COOH, p-TSA, CH₃SO₃H, HCl, H₂SO₄, HF, HBr, HBF₄. Besonders bevorzugt sind CH₃COOH und CH₃SO₃H. Bei der Durchführung des erfindungsgemäßen Verfahrensschritts 2 setzt man für 1 Mol des Benzoxazinones der Formel (IV) vorzugsweise 0,001 Mol bis 1,5 Mol, bevorzugt 0,01 Mol bis 1 Mol, besonders bevorzugt 0,01 bis 0,5 Mol, ganz besonders bevorzugt 0,01 Mol bis 0,3 Mol, insbesondere bevorzugt 0,1 Mol bis 0,3 Mol der Säure ein.

Der erfindungsgemäße Verfahrensschritt (2) wird im Allgemeinen unter Normaldruck durchgeführt. Alternativ ist es jedoch auch möglich, im Vakuum oder unter Überdruck im Autoklav zu arbeiten.

Die Reaktionszeit kann, in Abhängigkeit von der Ansatzgröße und der Temperatur, in einem Bereich zwischen 1 Stunde und mehreren Stunden gewählt werden.

Es ist auch möglich, die Schritte 1 und 2 ohne Zwischenisolierung von Benzoxazinonen durchzuführen.

### Herstellbeispiele

Die folgenden Herstellbeispiele illustrieren die Erfindung, ohne sie zu beschränken.

### Beispiel 1

Isomerengemisch von Methyl 1-(3-chloropyridin-2-yl)-3-{[5-(trifluoromethyl)-2H-tetrazol-2-yl]methyl}-1H-pyrazole-5-carboxylate (Hauptisomer) und Methyl 1-(3-chloropyridin-2-yl)-3-{[5-(trifluoromethyl)-1H-tetrazol-1-yl]methyl}-1H-pyrazole-5-carboxylate (Nebenkomponente).

2.86 g (0.01 mol) Methyl 3-(chlormethyl)-1-(3-chloropyridin-2-yl)-1H-pyrazole-5-carboxylate und 1,6 g (0.01 mol) Natrium 5-(trifluoromethyl)tetrazol-2-ide und 0.15 g KI in 50 ml Aceton wurden 12 Std bei 56 °C erhitzt. Die Salze wurden abfiltriert und Aceton im Vakuum entfernt. Man erhielt 34.59 g des Produktes als 9:1 Gemisch von beiden Isomeren.

### Analytische Charakterisierung

### Hauptisomer

¹H NMR (CD₃CN) δ: 8,52 (1H, d); 7.95 (1H,d), 7,45 (1H, dd); 7,10 (1H, s); 6.05 (2H,s); 3,75 (3H, s) ppm.

¹⁹F NMR -64.05 ppm.

### Nebenkomponente

¹⁹F NMR -61.46 ppm.

¹H NMR (CD₃CN) δ: 8,50 (1H, d); 7.90 (1H,d), 7,45 (1H, dd); 6,95 (1H, s); 5,80 (2H,s); 3,70 (3H, s) ppm.

### Beispiel 2

Isomerengemisch von 1-(3-chloropyridin-2-yl)-3-{[5-(trifluoromethyl)-2H-tetrazol-2-yl]methyl}-1H-pyrazole-5-carboxyl-Säure (Hauptisomer) und 1-(3-Chloropyridin-2-yl)-3-[5-(trifluoromethyl)-1H-tetrazol-1-yl]-1H-pyrazole-5-carboxyl-Säure (Nebenkomponente).

34.59 g des Gemisches aus dem Beispiel 1 wurden in 40 ml Methanol gelöst und 2 g NaOH als 10 % Lösung in Wasser wurden zugegeben. Das Gemisch wurde 3 Std. bei RT gerührt.

10 % iger HCl wurde zugegeben, um den pH der Lösung auf 3 einzustellen und das Produkt wurde mit Methyltert-butylether extrahiert. Nach der Entfernung des Lösemittels man erhält 34 g Produktes mit einer Reinheit von 99 %.

### Analytische Charakterisierung Hauptisomer 92 %

¹H NMR (CD₃CN) δ: 13,5 (b.s); 8,52 (1H, d); 8,2 (1H,d), 7,6 (1H, dd); 7,2 (1H, s); 6.25 (2H,s) ppm.

¹⁹F NMR -64.25 ppm.

### Beispiel 3

Isomerengemisch von 2-[1-(3-chloropyridin-2-yl)-3-{[5-(trifluoromethyl)-2H-tetrazol-2-yl]methyl}-1H-pyrazol-5-yl]-8-methyl-4-oxo-4H-3,1-benzoxazine-6-carbonitrile und 2-[1-(3-chloropyridin-2-yl)-3-{[5-(trifluoromethyl)-1H-tetrazol-1-yl]methyl}-1H-pyrazol-5-yl]-8-methyl-4-oxo-4H-3,1-benzoxazine-6-carbonitrile

18,83 g (50 mmol) von 1-(3-chloropyridin-2-yl)-3-{[5-(trifluoromethyl)-2H-tetrazol-2-yl]methyl}-1H-pyrazole-5-carboxylic Säure wurden in 60 ml Acetonitrile vorgelegt und das Gemisch auf 0°C gekühlt.

16,3 g b-Picoline und 8,16 g Methansülfonsäurechlorid wurden nacheinander zugegeben und das Gemisch 1 Std. bei 0°C nachgerührt. Anschließend wurden 10,1 g 8-Methyl-2,4-dioxo-1,4-dihydro-2H-3,1-benzoxazine-6-carbonitrile zugeben und das Reaktionsgemisch 10 Std. bei 50°C nachgerührt und auf 10°C abgekühlt. 50 ml Wasser wurden zu der Suspension zugegeben, der Niederschlag wurde ab filtriert und mit Wasser gewaschen. Man erhielt 23,9 g (Ausbeute 93 %) von Benzoxazinonen mit dem Isomerenverhältnis 91:9.

¹H NMR (DMF d₆) Haupisomer δ: 8,71 (1H,dd), 8,41(1H,d); 8,40 (1H,dd), 8,11 (1H, m); 7,84 (1H, dd), 7,68 (1H,s), 6.48 (2H,s), 1,83 (3H,s) ppm.

### Beispiel 4

Isomerengemisch von 1-(3-chloropyridin-2-yl)-N-[4-cyano-2-methyl-6-(methylcarbamoyl)-phenyl]-3-{[5-(trifluoromethyl)-2H-tetrazol-2-yl]methyl}-1H-pyrazole-5-carboxamide (Hauptisomer) und 1-(3-chloropyridin-2-yl)-N-[4-cyano-2-methyl-6-(methylcarbamoyl)phenyl]-3-[5-(trifluoro-methyl)-1H-tetrazol-1-yl]-1H-pyrazole-5-carboxamide (Nebenkomponente) im Verhältnis 92:8.

51,3 g des Isomerengemisches von 2-[1-(3-chloropyridin-2-yl)-3-{[5-(trifluoromethyl)-2H-tetrazol-2-yl]methyl}-1H-pyrazol-5-yl]-8-methyl-4-oxo-4H-3,1-benzoxazine-6-carbonitrile aus derm Beispiel 3 wurden bei 30°C in 200 ml Acetonitril suspendiert. Dann wurden 1,5 ml CH₃COOH zugegeben und danach 1,2 Äquivalent Methylamin (als Lösung in THF) bei 20°c zugetropft. Das Gemisch wurde 4 Std. bei 30°C nachgerührt, mit 100 ml Wasser verdünnt und die Suspension 3 Std. bei 50°C nachgerührt. Der Niederschlag wurde abfiltriert und getrocknet. Man erhielt 52 g (Ausbeute 93 %) des Produktes als weißen Feststoff mit einem Isomerenverhältnis von 92:8 und einer Reinheit von 97 % (w/w). Der Gehalt an 2-[1-(3-chloropyridin-2-yl)-3-{[5-(trifluoromethyl)-2H-tetrazol-2-yl]methyl}-1H-pyrazol-5-yl]-3,8-dimethyl-4-oxo-3,4-dihydroquinazoline-6-carbonitrile lag bei 0,1 %.

### Analytische Charakterisierung

### Hauptisomer

| H/C | δH/ppm | Mult. | rel. No. H | δC/ppm | Mult. | rel. No. C |
|---|---|---|---|---|---|---|
| 1 | - | - | - | 118.7 | Q | 1 |
| 2 | - | - | - | 156.1 | Q | 1 |
| 3 | 6.34 | S | 2 | 51.3 | T | 1 |
| 4 | - | - | - | 145.6 | S | 1 |
| 5 | 7.40 | S | 1 | 108.5 | D | 1 |
| 6 | - | - | - | 138.8 | S | 1 |
| 7 | - | - | - | 156.3 | S | 1 |
| 8 | 10.55 | S | 1 | - | - | - |
| 9 | - | - | - | 137.6 | S | 1 |
| 10 | - | - | - | 138.7 | S | 1 |
| 11 | - | - | - | 166.2 | S | 1 |
| 12 | 8.38 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.4 | S | 1 |
| 16 | - | - | - | 118.3 | S | 1 |
| 17 | 7.87 | D | 1 | 135.2 | D | 1 |
| 18 | - | - | - | 138.0 | S | 1 |
| 19 | 2.20 | S | 3 | 18.0 | Q | 1 |
| 20 | - | - | - | 149.1 | S | 1 |
| 21 | - | - | - | 128.0 | S | 1 |
| 22 | 8.16 | DD | 1 | 139.4 | D | 1 |
| 23 | 7.60 | DD | 1 | 126.7 | D | 1 |
| 24 | 8.48 | DD | 1 | 147.3 | D | 1 |

### Nebenkomponente

| H/C | δH/ppm | Mult. | rel. No. H | δC/ppm | Mult. | rel. No. C |
|---|---|---|---|---|---|---|
| 1 | - | - | - | 118.1 | Q | 1 |
| 2 | - | - | - | 145.9 | Q | 1 |
| 3 | 6.11 | S | 2 | 47.0 | T | 1 |
| 4 | - | - | - | 145.9 | S | 1 |
| 5 | 7.35 | S | 1 | 107.7 | D | 1 |
| 6 | - | - | - | 138.8 | S | 1 |
| 7 | - | - | - | 156.2 | S | 1 |
| 8 | 10.54 | S | 1 | - | - | - |
| 9 | - | - | - | 137.6 | S | 1 |
| 10 | - | - | - | 135.2 | S | 1 |
| 11 | - | - | - | 166.2 | S | 1 |
| 12 | 8.37 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.3 | S | 1 |
| 16 | - | - | - | 118.3 | S | 1 |
| 17 | 7.87 | D | 1 | 135.4 | D | 1 |
| 18 | - | - | - | 138.0 | S | 1 |
| 19 | 2.19 | S | 3 | 17.9 | Q | 1 |
| 20 | - | - | - | 149.1 | S | 1 |
| 21 | - | - | - | 128.1 | S | 1 |
| 22 | 8.14 | DD | 1 | 139.4 | D | 1 |
| 23 | 7.58 | DD | 1 | 126.7 | D | 1 |
| 24 | 8.47 | DD | 1 | 147.2 | D | 1 |

### Beispiel 5

Man arbeitet wie im Beispiel 4 beschrieben, nimmt jedoch 1 ml HCl (37 %). Man erhielt 51,2 g des Produktes mit einer Reinheit von 96 %. Der Anteil an 2-[1-(3-chloropyridin-2-yl)-3-{[5-(trifluoromethyl)-2H-tetrazol-2-yl]methyl}-1H-pyrazol-5-yl]-3,8-dimethyl-4-oxo-3,4-dihydroquinazoline-6-carbonitrile betrug 0,3 %.

### Beispiel 6

Man arbeitet wie im Beispiel 5 beschrieben, jedoch ohne CH₃COOH zu verwenden. Man erhielt 50 g des Produktesmit einer Reinheit von 92 %. Der Anteil an 2-[1-(3-chloropyridin-2-yl)-3-{[5-(trifluoromethyl)-2H-tetrazol-2-yl]methyl}-1H-pyrazol-5-yl]-3,8-dimethyl-4-oxo-3,4-dihydroquinazoline-6-carbonitrile lag bei 3 %.

## Patentansprüche

1. Verfahren zur Herstellung von Verbindungen der allgemeinen Formel (I) 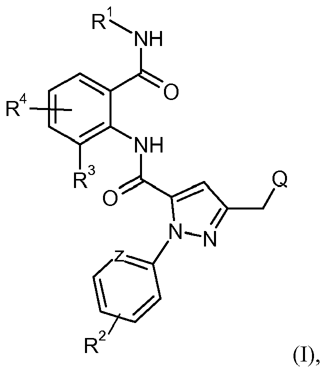 in welcher
R¹, R³ unabhängig voneinander für Wasserstoff, für gegebenenfalls einfach oder mehrfach, gleich oder verschieden durch Halogen oder Nitro substituiertes C₁-C₆-Alkyl, C₁-C₆-Alkoxy, C₂-C₆-Alkenyl, C₂-C₆-Alkinyl oder C₃-C₆-Cycloalkyl steht,
R² für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalkenyl, C₁-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Halo-alkylsulfinyl, C₁-C₄-Haloalkylsulfonyl, Halogen, Cyano, Nitro, Alkylamino, Dialkylamino, Cycloalkylamino oder C₁-C₆-Trialkylsilyl steht,
R⁴ für Wasserstoff, Halogen, Cyano, Nitro, C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, SF₅, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₄-Alkoxy)imino, (C₁-C₄-Alkyl)(C₁-C₄-Alkoxy)imino, (C₁-C₄-Haloalkyl)(C₁-C₄-Cyano, Nitro, Alkoxy)imino oder C₃-C₆-Trialkylsilyl steht,
Q für einen durch R⁵ einfach substituierten Tetrazolring steht, bevorzugt für einen durch R⁵ einfach substituierten Tetrazolring steht, ausgewählt aus der Gruppe bestehend aus 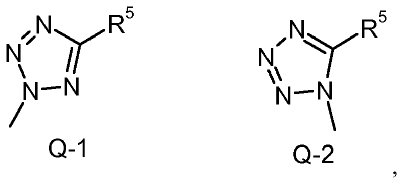
R⁵ für C₁-C₅-Alkyl steht, welches 1 bis 3 fach durch Halogen substituiert sein kann,
Z für CH und N steht,
die Verbindungen der allgemeinen Formel (I) außerdem N-Oxide und Salze umfassen,
**dadurch gekennzeichnet, dass** man tetrazol-substituierte Pyrazolsäuren der Formel (II) 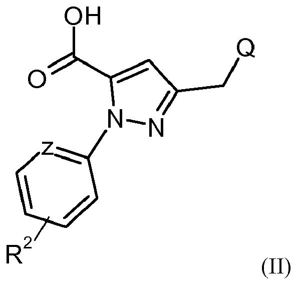 in welcher R², Q und Z oben angegebenen Bedeutungen haben
mit Isatoanhydriden der Formel (III) 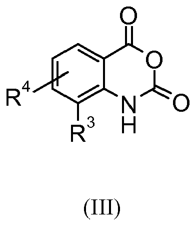 in welcher
R³,R⁴ die oben angegebenen Bedeutungen haben,
umsetzt zu Verbindungen der Formel (IV) 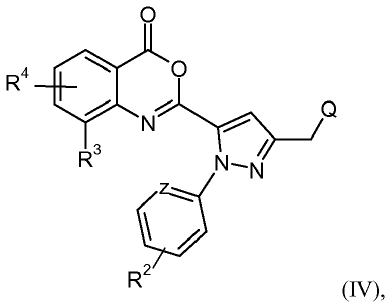 in welcher R², R³, R⁴, Q und Z oben angegebenen Bedeutungen haben,
und diese Verbindungen der allgemeinen Formel (IV) in Gegenwart einer Säure mit Aminen der allgemeinen Formel (V)
R¹NH₂ (V)
in welcher R¹ die oben angegebenen Bedeutungen hat,
umsetzt zu Verbindungen der Formel (I).

2. Verfahren zur Herstellung von Verbindungen der Formel (I) gemäß Anspruch 1, **dadurch gekennzeichnet dass**
R¹, R³ unabhängig voneinander für (C₁-C₅)-Alkyl steht,
R² für Halogen oder C₁-C₆-Alkyl steht,
R⁴ für Wasserstoff, Chlor oder Cyano steht,
Q für einen durch R⁵ einfach substituierten Tetrazolring steht, ausgewählt aus der Gruppe bestehend aus 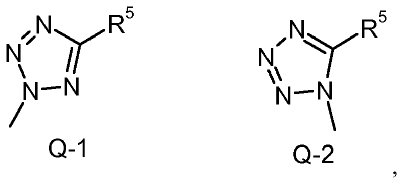
R⁵ für (C₁-C₃)Perfluoralkyl steht,
Z für N steht.

3. Verfahren zur Herstellung von Verbindungen der Formel (I) gemäß einem der Ansprüche 1 oder 2, **dadurch gekennzeichnet, dass**
R¹, R³ unabhängig voneinander für Methyl, Ethyl oder tert-Butyl steht,
R² für Fluor oder Chlor steht,
R⁴ für Chlor oder Cyano steht,
Q für Q-1 oder Q-2 steht,
R⁵ für CF₃ oder C₂F₅ steht,
Z für N steht.

4. Verfahren zur Herstellung von Verbindungen der Formel (I) gemäß einem der Ansprüche 1 bis 3, **dadurch gekennzeichnet dass** R⁵ für CF₃ steht.

5. Verfahren zur Herstellung von Verbindungen der Formel (I) gemäß einem der Ansprüche 1 bis 4, **dadurch gekennzeichnet, dass** R² für Chlor, R³ für Methyl und R⁴ für Cyano steht.

6. Verfahren zur Herstellung von Verbindungen der Formel (I) gemäß einem der Ansprüche 1 bis 5, **dadurch gekennzeichnet, dass** das Verhältnis von Verbindungen der Formel (I), in welchen Q für Q-1 steht zu Verbindungen der Formel (I), in welchen Q für Q-2 steht, 90:10 bis 96:4 beträgt.

7. Verfahren zur Herstellung von Verbindungen der Formel (I) gemäß einem der Ansprüche 1 bis 6, **dadurch gekennzeichnet, dass** bei Umsetzung der Verbindungen der Formel (IV) mit den Verbindungen der Formel (V) auf 1 Mol Verbindungen der Formel (IV) 0,001 bis 1,5 Mol einer organischen oder anorganischen Säure zugegeben werden.

8. Verfahren zur Herstellung von Verbindungen der Formel (I) gemäß einem der Ansprüche 1 bis 7, **dadurch gekennzeichnet, dass** bei Umsetzung der Verbindungen der Formel (IV) mit den Verbindungen der Formel (V) auf 1 Mol Verbindungen der Formel (IV) 0,01 bis 0,3 Mol CF₃COOH zugegeben werden.

9. Verfahren zur Herstellung von Verbindungen der Formel (I) gemäß einem der Ansprüche 1 bis 8, **dadurch gekennzeichnet, dass** die Verbindungen der Formel (IV) bei einer Reaktionstemperatur von 0°C bis +100°C mit den Verbindungen der Formel (V) zu Anthranilsäurediamid-Derivaten der Formel (I) umgesetzt werden.

## Claims

1. Process for preparing compounds of the general formula (I) 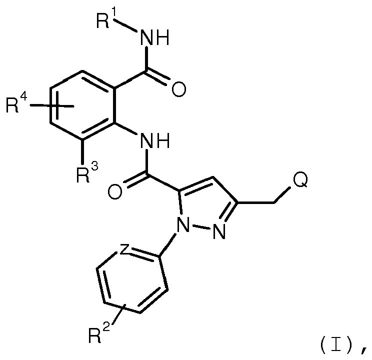 in which
R¹, R³ independently of one another represent hydrogen, or represent C₁-C₆-alkyl, C₁-C₆-alkoxy, C₂-C₆-alkenyl, C₂-C₆-alkynyl or C₃-C₆-cycloalkyl which are each optionally mono- or polysubstituted by identical or different halogen or nitro substituents,
R² represents C₁-C₆-alkyl, C₃-C₆-cycloalkyl, C₁-C₆-haloalkyl, C₁-C₆-halocycloalkyl, C₂-C₆-alkenyl, C₂-C₆-haloalkenyl, C₂-C₆-alkynyl, C₂-C₆-haloalkynyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, halogen, cyano, nitro, alkylamino, dialkylamino, cycloalkylamino or C₃-C₆-trialkylsilyl,
R⁴ represents hydrogen, halogen, cyano, nitro, C₁-C₄-alkyl, C₁-C₄-haloalkyl, C₂-C₆-alkenyl, C₂-C₆-haloalkenyl, C₂-C₆-alkynyl, C₁-C₄-alkoxy, C₁-C₄-haloalkoxy, SF₅, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, di-(C₁-C₄-alkyl)amino, C₃-C₆-cycloalkylamino, (C₁-C₄-alkoxy)imino, (C₁-C₄-alkyl) (C₁-C₄-alkoxy) imino, (C₁-C₄-haloalkyl) (C₁-C₄-cyano, nitro, alkoxy)imino or C₃-C₆-trialkylsilyl,
Q represents a tetrazole ring which is monosubstituted by R⁵, preferably a tetrazole ring which is monosubstituted by R⁵ and selected from the group consisting of 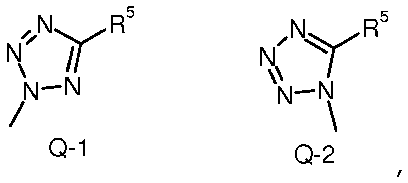
R⁵ represents C₁-C₅-alkyl which may be mono- to trisubstituted by halogen,
Z represents CH or N,
the compounds of the general formula (I) furthermore include N-oxides and salts,
**characterized in that** tetrazole-substituted pyrazole acids of the formula (II) 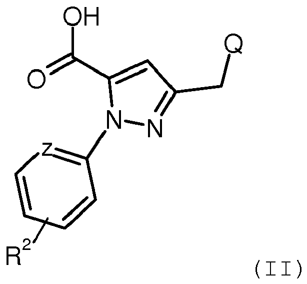 in which R², Q and Z have the meanings given above
are reacted with isatoic anhydrides of the formula (III) 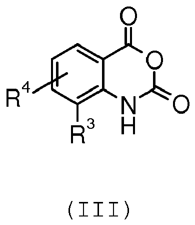 in which
R³, R⁴ have the meanings given above,
to give compounds of the formula (IV) 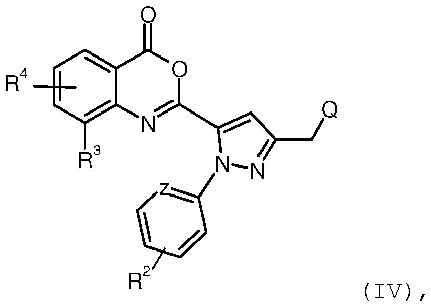 in which R², R³, R⁴, Q and Z have the meanings given above,
and these compounds of the general formula (IV) are reacted in the presence of an acid with amines of the general formula (V)
R¹NH₂ (V)
in which R¹ has the meanings given above,
to give compounds of the formula (I).

2. Process for preparing compounds of the formula (I) according to Claim 1, **characterized in that**
R¹, R³ independently of one another represent (C₁-C₅)-alkyl,
R² represents halogen or C₁-C₆-alkyl,
R⁴ represents hydrogen, chlorine or cyano,
Q represents a tetrazole ring which is monosubstituted by R⁵ and selected from the group consisting of 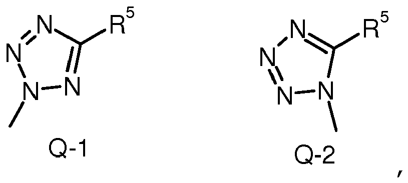
R⁵ represents (C₁-C₃)-perfluoroalkyl,
Z represents N.

3. Process for preparing compounds of the formula (I) according to Claim 1 or 2, **characterized in that**
R¹, R³ independently of one another represent methyl, ethyl or tert-butyl,
R² represents fluorine or chlorine,
R⁴ represents chlorine or cyano,
Q represents Q-1 or Q-2,
R⁵ represents CF₃ or C₂F₅,
Z represents N.

4. Process for preparing compounds of the formula (I) according to any of Claims 1 to 3, **characterized in that** R⁵ represents CF₃.

5. Process for preparing compounds of the formula (I) according to any of Claims 1 to 4, **characterized in that** R² represents chlorine, R³ represents methyl and R⁴ represents cyano.

6. Process for preparing compounds of the formula (I) according to any of Claims 1 to 5, **characterized in that** the ratio of compounds of the formula (I) in which Q represents Q-1 to compounds of the formula (I) in which Q represents Q-2 is from 90:10 to 96:4.

7. Process for preparing compounds of the formula (I) according to any of Claims 1 to 6, **characterized in that**, when the compounds of the formula (IV) are reacted with the compounds of the formula (V), from 0.001 to 1.5 mol of an organic or inorganic acid are added per 1 mol of compounds of the formula (IV).

8. Process for preparing compounds of the formula (I) according to any of Claims 1 to 7, **characterized in that**, when the compounds of the formula (IV) are reacted with the compounds of the formula (V), from 0.01 to 0.3 mol of CF₃COOH are added per 1 mol of compounds of the formula (IV).

9. Process for preparing compounds of the formula (I) according to any of Claims 1 to 8, **characterized in that** the compounds of the formula (IV) are reacted at a reaction temperature of from 0°C to +100°C with the compounds of the formula (V) to give anthranilic acid diamide derivatives of the formula (I).

## Revendications

1. Procédé pour la préparation de composés de formule générale (I) 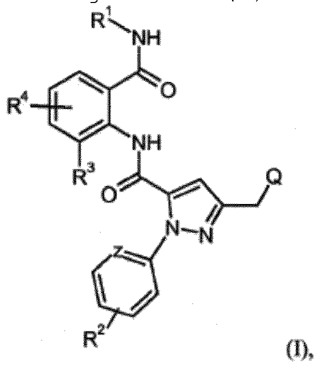 dans laquelle
R¹, R³ représentent, indépendamment l'un de l'autre, hydrogène, C₁-C₆-alkyle, C₁-C₆-alcoxy, C₂-C₆-alcényle, C₂-C₆-alcynyle ou C₃-C₆-cycloalkyle, le échéant monosubstitués ou polysubstitués, de manière identique ou différente par halogène ou nitro,
R² représente C₁-C₆-alkyle, C₃-C₆-cycloalkyle, C₁-C₆-halogénoalkyle, C₁-C₆-halogénocycloalkyle, C₂-C₆-alcényle, C₂-C₆-halogénoalcényle, C₂-C₆-alcynyle, C₂-C₆-halogénoalcynyle, C₁-C₄-alcoxy, C₁-C₄-halogénoalcoxy, C₁-C₄-alkylthio, C₁-C₄-alkylsulfinyle, C₁-C₄-alkylsulfonyle, C₁-C₄-halogénoalkylthio, C₁-C₄-halogénoalkylsulfinyle, C₁-C₄-halogénoalkylsulfonyle, halogène, cyano, nitro, alkylamino, dialkylamino, cycloalkylamino ou C₃-C₆-trialkylsilyle,
R⁴ représente hydrogène, halogène, cyano, nitro, C₁-C₄-alkyle, C₁-C₄-halogénoalkyle, C₂-C₆-alcényle, C₂-C₆-halogénoalcényle, C₂-C₆-alcynyle, C₁-C₄-alcoxy, C₁-C₄-halogénoalcoxy, SF₅, C₁-C₄-alkylthio, C₁-C₄-alkylsulfinyle, C₁-C₄-alkylsulfonyle, C₁-C₄-halogénoalkylthio, C₁-C₄-halogénoalkylsulfinyle, C₁-C₄-halogénoalkylsulfonyle, C₁-C₄-alkylamino, di-(C₁-C₄-alkyl)amino, C₃-C₆-cycloalkylamino, (C₁-C₄-alcoxy)imino, (C₁-C₄-alkyl) (C₁-C₄-alcoxy)imino, (C₁-C₄-halogénoalkyl) (C₁-C₄-cyano, nitro, alcoxy)imino ou C₃-C₆-trialkylsilyle,
Q représente un cycle tétrazole monosubstitué par R⁵, de préférence un cycle tétrazole monosubstitué par R⁵ choisi dans le groupe constitué par 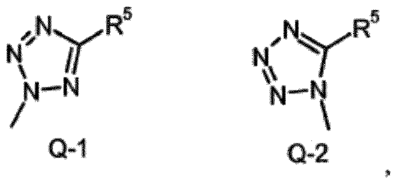
R⁵ représente C₁-C₅-alkyle, qui peut être monosubstitué à trisubstitué par halogène,
Z représente CH et N, les composés de formule générale (I) comprenant en outre des N-oxydes et des sels, **caractérisé en ce qu'**on transforme des acides pyrazoliques substitués par tétrazole de formule (II) 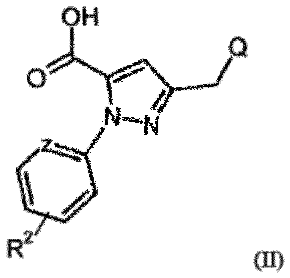 dans laquelle R², Q et Z présentent les significations indiquées ci-dessus,
avec des isatoanhydrides de formule (III) 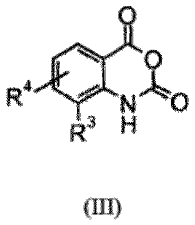 dans laquelle R³ et R⁴ présentent les significations indiquées ci-dessus,
en composés de formule (IV) 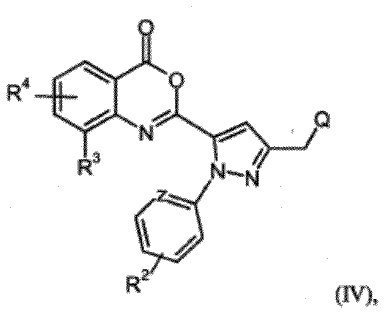 dans laquelle R², R³, R⁴, Q et Z présentent les significations indiquées ci-dessus,
et on transforme ces composés de formule générale (IV) en présence d'un acide avec des amines de formule générale (V)
R¹NH₂ (V)
dans laquelle R¹ présente les significations indiquées ci-dessus, en composés de formule (I).

2. Procédé pour la préparation de composés de formule (I) selon la revendication 1, **caractérisé en ce que**
R¹, R³ représentent, indépendamment l'un de l'autre, (C₁-C₅)-alkyle.
R² représente halogène ou C₁-C₆-alkyle,
R⁴ représente hydrogène, chlore ou cyano,
Q représente un cycle tétrazole monosubstitué par R⁵ choisi dans le groupe constitué par 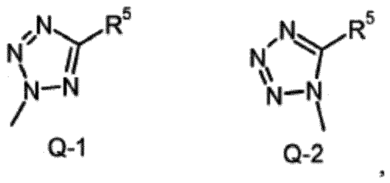
R⁵ représente (C₁-C₃)-perfluoroalkyle,
Z représente N.

3. Procédé pour la préparation des composés de formule (I) selon l'une quelconque des revendications 1 ou 2, **caractérisé en ce que**
R¹, R³ représentent, indépendamment l'un de l'autre, méthyle, éthyle ou tert-butyle,
R² représente fluor ou chlore,
R⁴ représente chlore ou cyano,
Q représente Q-1 ou Q-2,
R⁵ représente CF₃ ou C₂F₅,
Z représente N.

4. Procédé pour la préparation de composés de formule (I), selon l'une quelconque des revendications 1 à 3, **caractérisé en ce que** R⁵ représente CF₃.

5. Procédé pour la préparation de composés de formule (I) selon l'une quelconque des revendications 1 à 4, **caractérisé en ce que** R² représente chlore, R³ représente méthyle et R⁴ représente cyano.

6. Procédé pour la préparation de composés de formule (I) selon l'une quelconque des revendications 1 à 5, **caractérisé en ce que** le rapport des composés de formule (I) dans laquelle Q représente Q-1 aux composés de formule (I) dans laquelle Q représente Q-2 vaut 90:10 à 96:4.

7. Procédé pour la préparation de composés de formule (I) selon l'une quelconque des revendications 1 à 6, **caractérisé en ce que**, lors de la transformation des composés de formule (IV) avec les composés de formule (V), on ajoute, pour 1 mole de composés de formule (IV), 0,001 à 1,5 mole d'un acide organique ou inorganique.

8. Procédé pour la préparation de composés de formule (I) selon l'une quelconque des revendications 1 à 7, **caractérisé en ce que**, lors de la transformation des composés de formule (IV) avec les composés de formule (V), on ajoute, pour 1 mole de composés de formule (IV), 0,01 à 0,3 mole de CF₃COOH.

9. Procédé pour la préparation de composés de formule (I) selon l'une quelconque des revendications 1 à 8, **caractérisé en ce que** les composés de formule (IV) sont transformés à une température de 0°C à +100°C avec les composés de formule (V) en dérivés de diamide de l'acide anthranilique de formule (I).
